# EUROPÄISCHE PATENTSCHRIFT

(11) **EP 1 124 782 B1**
(45) Veröffentlichungstag und Bekanntmachung des Hinweises auf die Patenterteilung: **02.01.2004**
(21) Anmeldenummer: 99949006.3
(22) Anmeldetag: 16.10.1999
(51) Int. Cl.: C07C 51/487, C07B 57/00, C07C 59/48, C07C 59/64

(54) **VERFAHREN ZUR RACEMATSPALTUNG VON 2-HYDROXYPROPIONSÄUREN**
METHOD FOR RACEMATE SPLITTING OF 2-HYDROXYPROPIONIC ACIDS
PROCEDE DE FISSION RACEMIQUE D'ACIDES 2-HYDROXYPROPIONIQUES

(30) Priorität: 30.10.1998 DE 19850301
(43) Veröffentlichungstag der Anmeldung: 22.08.2001
(73) Patentinhaber: Abbott GmbH & Co. KG, 65205 Wiesbaden (DE)
(72) Erfinder: BERNARD, Harald, D-67157 Wachenheim (DE); RIECHERS, Hartmut, D-67435 Neustadt (DE)
(74) Vertreter: Grünecker, Kinkeldey, Stockmair & Schwanhäusser Anwaltssozietät
(86) Internationale Anmeldenummer: PCT/EP1999/007858
(87) Internationale Veröffentlichungsnummer: WO 2000/026170

(56) Entgegenhaltungen:
- EP-A- 0 915 080
- WO-A-93/13075
- WO-A-96/11914
- KINBARA K ET AL: "(2-Naphthyl)glycolic acid: a tailored resolving agent for p-substituted 1-arylethylamines" TETRAHEDRON: ASYMMETRY, Bd. 9, Nr. 13, 3. Juli 1998 (1998-07-03), Seiten 2219-2222, XP004129024 ISSN: 0957-4166

## Beschreibung

Die vorliegende Erfindung betrifft ein Verfahren zur Racematspaltung von 2-Hydroxypropionsäuren durch Umsetzen der racemischen Säure mit einer optisch aktiven Base und anschließender Abtrennung eines diastereomeren Salzes aus Säure und Base. 2-Hydroxypropionsäuren sind wichtige Zwischenprodukte zur Synthese von Pflanzenschutzmitteln und Pharmazeutika. Da die Wirkung solcher Stoffe häufig nur auf ein Enantiomeres zurückzuführen ist, ist es wünschenswert, diese Wirkstoffe in hoher optischer Reinheit herzustellen. Es empfiehlt sich deshalb, die Synthese so zu planen, daß racemische Zwischenprodukte in ihre Enantiomeren getrennt werden um anschließend enantiomerenreine Endprodukte zu erhalten.

In WO 96/11914 werden 2-heterozyklisch substituierte Propionsäuren als potente Endothelinrezeptorantagonisten beschrieben zur Behandlung von Herz-Kreislauferkrankungen beschrieben. Diese Wirkstoffe werden unter Verwendung von 2-Hydroxypropionsäuren als zwischenprodukte synthetisiert.

In WO 96/11914 wird die Herstellung im Labormaßstab von (S)-2-Hydroxy-3-methoxy-3,3-diphenylpropionsäure durch Racematspaltung mit L-Prolinmethylester (Beispiel 10) und (S)-1-(4-Nitrophenyl)-ethylamin (Beispiel 11) beschrieben. Es wird damit eine Ausbeute von 35% bezogen auf das Racemat sowie eine optische Reinheit von 99,8% erreicht.

Es hat sich jedoch gezeigt, daß bei der Übertragung dieses beschriebenen Reaktionsschrittes auf einen größeren Maßstab (mehrere kg bis 100 kg) zusätzliche Arbeitsschritte notwendig wurden, um eine hohe optische Reinheit zu gewährleisten. Das diastereomere Salz aus (S)-2-Hydroxypropionsäure und (S)-1-(4-Nitrophenyl)ethylamin kristallisiert schlecht und läßt sich deshalb auch nicht gut abfiltrieren, so daß ein Teil der Mutterlauge mit dem abzutrennenden Enantiomeren im Kristallisat verbleibt. Nur wenn das Kristallisat zusätzlich im Kessel mit frischem Lösungsmittel aufgerührt wurde und wenn die nochmals abfiltrierten Kristalle ausgiebig nachgewaschen worden waren, wurde die geforderte optische Reinheit erhalten.

Es bestand daher die Aufgabe, ein Verfahren zur Racematspaltung bereitzustellen, das nicht die oben genannten Nachteile besitzt, sondern sich leicht im technischen Maßstab durchführen läßt.

Gefunden wurde ein Verfahren zur Racematspaltung von 2-Hydroxypropionsäuren durch Umsetzen der racemischen Säure mit einer optisch aktiven Base und anschließender Abtrennung eines diastereomeren Salzes aus Säure und Base, das dadurch gekennzeichnet ist, daß als optisch aktive Base 1-(4-Chlorphenyl)ethylamin verwendet wird.

Die Herstellung von 2-Hydroxypropionsäuren ist dem Fachmann aus Handbüchern der organischen Synthese geläufig und beispielsweise in WO 96/11914 beschrieben.

Das erfindungsgemäße Verfahren eignet sich besonders zur Racematspaltung von 2-Hydroxypropionsäuren, die an der 3-Position ein oder mehrfach substituiert sind, insbesondere von 2-Hydroxypropionsäuren, die an der 3-Position ein bis zwei Aryl-, bevorzugt Phenylreste tragen.

Eine besonders bevorzugte Ausführungsform des erfindungsgemäßen Verfahrens ist die Racematspaltung von 2-Hydroxy-3-methoxy-3,3-diphenylpropionsäure und 2-Hydroxy-3-methyl-3,3-diphenylpropionsäure.

Das erfindungsgemäße Verfahren wird üblicherweise so durchgeführt, daß eine Lösung von x Mol der racemischen 2-Hydroxypropionsäure, ggf. unter Erwärmen, mit etwa 0,5 - 0,6 x Mol optisch aktivem 1-(4-Chlorphenyl)ethylamin versetzt wird und anschließend eines der gebildeten diastereomeren Salze abgetrennt wird.

In einer bevorzugten Ausführungsform gibt man zu der Lösung Impfkristalle des abzutrennenden diastereomeren Salzes zu und läßt die Lösung langsam abkühlen, wodurch eine gute Kristallisation erreicht wird. Man kann die Impfkristalle zugeben nachdem die gesamte Menge an optisch aktiver Base zugesetzt worden ist oder aber auch schon früher, wenn erst ein Teil, bevorzugt etwa die Hälfte, der benötigten Base zugegeben worden ist.

Falls das andere Enantiomer der 2-Hydroxypropionsäure gewünscht wird, kann dies auch anschließend aus der Mutterlauge isoliert werden.

Ein bevorzugtes Lösungsmittel für die Racemattrennung ist ein Gemisch aus Alkohol und Ether, insbesondere aus Methanol und Methyl-tert.- Butylether (MTB). Für die Racematspaltung von 3-Hydroxy-3-methyl-3,3-diphenylpropionsäure wird bevorzugt ein Alkohol, insbesondere Isopropanol, verwendet.

Das erfindungsgemäße Verfahren besitzt die Vorteile, daß es in sehr guter Ausbeute enantiomerenreine 2-Hydroxypropionsäure liefert und problemlos in den technischen Maßstab übertragen werden kann. Die Kristalle des diastereomeren Salzes aus 2-Hydroxypropionsäure und 1-(4-Chlorphenyl)ethylamin setzen sich überraschend gut in der Mutterlauge ab und lassen sich daher problemlos durch Filtration abtrennen. Ein aufwendiges Nachwaschen des Kristallisats zur Erreichung der gewünschten Enantiomerenreinheit ist nicht nötig. Dies ist ein wesentlicher Vorteil gegenüber dem diastereomeren Salz aus 2-Hydroxypropionsäure und 1-(4-Nitrophenyl)ethylamin. Somit lassen sich aufwendige Umfüllarbeiten, Rühr- und Nachwaschschritte einsparen, wodurch Energieeinsatz und Lösungsmittelmenge verringert werden und die Raum-Zeit-Ausbeute einer gegebenen Anlage wesentlich gesteigert werden können.
Das folgende Beispiel veranschaulicht das erfindungsgemäße Verfahren in einem technischen Maßstab.

### Beispiel 1

### Herstellung von (S)-p-Chlorphenylethylammonium-(S)-2-Hydroxy-3-methoxy-3,3-diphenylpropionat durch Racematspaltung

Eine Lösung von 129,0 kg (474 Mol) 2-Hydroxy-3-methoxy-3,3-diphenylpropionsäure in 650 l MTB und 650 l Methanol wird auf 55°C erwärmt und unter Rühren und Einhalten der 55°C mit 37,0 kg (237 Mol) (S)-p-Chlorphenylethylamin versetzt. Nach Zugabe der halben Menge des Amins wird mit (S)-p-Chlorphenylethylammonium-(S)-2-Hydroxy-3-methoxy-3,3-diphenylpropionat angeimpft, wobei die Kristallisation beginnt. Ist die Gesamtmenge Amin zugegeben, wird noch eine Stunde bei 55°C nachgerührt und dann mit einer Abkühlrate von 10°C pro Stunde auf 3°C abgekühlt.

Die entstandene Suspension wird über einen Einschichtenfilter abfiltriert und mit 300 l auf 3°C abgekühltem MTB nachgewaschen. Das Produkt wird auf dem Filter unter Beheizung des Mantels auf 40°C im Stickstoffstrom getrocknet.

Ausbeute: 73,9 kg (172,7 Mol) entspricht 36,4% d.Th. (bezogen auf Racemat)

HPLC: 99,8 %

Chir. HPLC: > 99,95 %

### Beispiel 2

Eine Lösung von 35,5 kg (134,4 mol) 2-Hydroxy-3-methyl-3,3-diphenylpropionsäure in 155 kg Isopropanol wird 2 h auf 75 bis 80°C erhitzt und mit 13,75 kg 1-(4-Chlorphenyl)ethylamin versetzt. Nach Zugabe von Impfkristallen wird noch weitere 2 h bei Rückfluß erhitzt und in Schritten von 5°C/h auf Raumtemperatur abgekühlt. Die entstandene Suspension wird über einen Einschichtfilter abgesaugt und zweimal mit 12 kg gekühltem Isopropanol gewaschen. Das Produkt wird im Filter unter Beheizung des Mantels auf 40°C im Stickstoffstrom getrocknet.
Gegebenenfalls wird das Produkt aus Isopropanol (Isopropanolmenge [L] = Masse des falschen Enantiomers [kg]/0,027 kg/L) umkristallisiert.

Ausbeute: 21,1 kg (51,2 Mol), Reinheit: 99,2 % entspricht 38 % bez. auf Racemat, ee = 94 %

## Patentansprüche

1. Verfahren zur Racematspaltung von 2-Hydroxypropionsäuren durch Umsetzen der racemischen Säure mit einer optisch aktiven Base und anschließender Abtrennung eines diastereomeren Salzes aus Säure und Base, **dadurch gekennzeichnet, daß** als optisch aktive Base 1-(4-Chlorphenyl)ethylamin verwendet wird.

2. Verfahren nach Anspruch 1, **dadurch gekennzeichnet, daß** es zur Racematspaltung von 2-Hydroxy-3-methoxy-3,3-diphenylpropionsäure eingesetzt wird.

3. Verfahren nach Anspruch 1, **dadurch gekennzeichnet, daß** es zur Racematspaltung von 2-Hydroxy-3-methyl-3,3-diphenylpropionsäure eingesetzt wird.

4. Verfahren nach Anspruch 1, **dadurch gekennzeichnet, daß** (S)-1-(4-Chlorphenyl)ethylamin als optisch aktive Base eingesetzt wird.

5. Verfahren nach Anspruch 2, **dadurch gekennzeichnet, daß** als Lösungsmittel ein Gemisch aus Methanol und Methyl-tert.-Butylether verwendet wird.

6. Verfahren nach Anspruch 3, **dadurch gekennzeichnet, daß** als Lösungsmittel Isopropanol verwendet wird.

## Claims

1. Process for the racemate resolution of 2-hydroxypropionic acids by reacting the racemic acid with an optically active base and subsequently separating off a diastereomeric salt of acid and base, **characterised in that** 1-(4-chlorophenyl)ethylamine is used as the optically active base.

2. Process according to Claim 1, **characterised in that** it is utilised for the racemate resolution of 2-hydroxy-3-methoxy-3,3-diphenylpropionic acid.

3. Process according to Claim 1, **characterised in that** it is utilised for the racemate resolution of 2-hydroxy-3-methyl-3,3-diphenylpropionic acid.

4. Process according to Claim 1, **characterised in that** (S)-1-(4-chlorophenyl)ethylamine is utilised as the optically active base.

5. Process according to Claim 2, **characterised in that** a mixture of methanol and methyl tert.-butyl ether is used as a solvent.

6. Process according to Claim 3, **characterised in that** isopropanol is used as a solvent.

## Revendications

1. Procédé de résolution racémique d'acides 2-hydroxypropioniques par réaction de l'acide racémique avec une base optiquement active et une séparation postérieure d'un sel diastéréoisomère d'une acide et d'une base, caractérisé en ce qu"on utilise comme base optiquement active la 1-(4-chlorophényl)éthylamine,

2. Procédé selon la revendication 1, **caractérisé en ce qu'**on utilise pour la résolution racémique de l'acide 2-hydroxy-3-méthoxy-3,3-diphénylpropionique.

3. Procédé selon la revendication 1, **caractérisé en ce qu'**on utilise pour la résolution racémique de l'acide 2-hydroxy-3-méthyl-3,3-diphénylpropionique.

4. Procédé selon la revendication 1, **caractérisé en ce qu'**on utilise comme base optiquement active la (S)-1-(4-chlorophényl)éthylamine.

5. Procédé selon la revendication 2, **caractérisé en ce qu'**on utilise comme solvant un mélange de méthanol et d'éther méthyl-tert.-butylique.

6. Procédé selon la revendication 3, **caractérisé en ce qu'**on utilise comme solvant de l'isopropanol.
